# EUROPEAN PATENT SPECIFICATION

(11) **EP 2 664 268 B1**
(45) Date of publication and mention of the grant of the patent: **27.04.2016**
(21) Application number: 12802886.7
(22) Date of filing: 16.05.2012
(51) Int. Cl.: A61B 1/00, A61B 1/04, A61B 1/045, A61B 1/06, A61B 1/31, H04N 5/225, H04N 5/232, H04N 5/235

(54) **MEDICAL INSTRUMENT**
MEDIZINISCHES INSTRUMENT
INSTRUMENT MÉDICAL

(30) Priority: 21.06.2011 JP 2011137752
(43) Date of publication of application: 20.11.2013
(73) Proprietor: Olympus Corporation, Shibuya-ku Tokyo (JP)
(72) Inventor: TAKEI Shunji, Tokyo 151-0072 (JP); ONO Wataru, Tokyo 151-0072 (JP)
(74) Representative: Gunzelmann, Rainer
(86) International application number: PCT/JP2012/062494
(87) International publication number: WO 2012/176561

(56) References cited:
- EP-A1- 2 082 680
- EP-A2- 2 209 303
- JP-A- 2004 525 573
- JP-A- 2005 057 471
- JP-A- 2005 057 471
- JP-A- 2007 313 170
- JP-A- 2009 172 287
- JP-A- 2010 068 992
- US-A- 5 827 190
- US-A1- 2007 276 184
- US-B1- 6 972 791

## Description

### Technical Field

The present invention relates to a medical instrument suitable for fluorescence observation.

### Background Art

Recently, attention has been focused on a cancer diagnosis technique using a molecular target drug. The technique is such that, after spraying or injecting a fluorescence probe (fluorescence medicine) targeting a biological protein which specifically appears in a cancer cell on or into a target site of a living body, it is determined whether cancer exists or not on the basis of fluorescence emitted at the target site. The technique is useful for an early detection of cancer in a field of digestive tracts.

In an endoscope apparatus, diagnosis using the technique is possible. That is, by radiating excitation light from a light source apparatus to an object via an endoscope insertion portion and catching fluorescence from fluorescence medicine accumulated in cancer by an image pickup section provided in the insertion portion, diagnosis of existence of cancer or quality diagnosis, such as diagnosis of malignant degree, is performed.

For example, in diagnosis of a large intestine mucosa, a fluorescent area, which is an area where fluorescence medicine accumulates (a marker accumulation area), is found by inserting the endoscope insertion portion into a large intestine and observing the large intestine mucosa while moving the insertion portion.

However, since the marker accumulation area is small, in such cases as when the moving speed of the endoscope insertion portion is high and when the movement of the object is drastic, the marker accumulation area enters the field of view of the image pickup section only for a moment in some cases. This results in increase of the risk of missing the marker accumulation area.

To avoid such a risk, a method is conceivable for improving followability of a motion picture by increasing the frame rate of the image pickup section in a scene where relative velocity between an object and the endoscope is high, to thereby decrease the risk of overlooking. For example, an endoscope system capable of adjusting the frame rate is disclosed in Japanese Patent Application Laid-Open Publication No. 2007-313170 and in US2007276184 A1. However, increasing the frame rate has caused a problem that the exposure time for detecting fluorescence is shortened, the picked-up image is darkened, and the visibility of the fluorescent area is deteriorated. The same problem occurs not only during fluorescence observation but also when performing special-light observations such as narrow band light observation.

An objective of the present invention is to provide a medical instrument capable of adjusting the frame rate while maintaining the brightness of the picked-up image, and of reducing overlook of the site to be observed during special-light observation.

### Disclosure of Invention

### Means for Solving the Problem

This objective is achieved by the subject-matter of claim 1.

### Brief Description of the Drawings

Fig. 1 is a block diagram showing a medical instrument according to a first embodiment of the present invention;
Fig. 2 is a diagram showing a state of fluorescence observation;
Fig. 3 is a diagram showing each frame of a moving image 71 obtained by the fluorescence observation in Fig. 2;
Fig. 4 is a diagram for illustrating a relationship between reading of an image signal from an image pickup section and timings of radiating illuminating light;
Fig. 5 is a diagram for illustrating pixels read from the image pickup section;
Fig. 6 is a schematic circuit diagram showing a configuration of a CMOS sensor;
Fig. 7 is a timing chart for illustrating an operation of the first embodiment of the present invention;
Fig. 8 is a flowchart for illustrating the operation of the first embodiment of the present invention;
Fig. 9 is a timing chart for illustrating a second embodiment of the present invention;
Fig. 10 is a block diagram showing a third embodiment of the present invention; and
Fig. 11 is a block diagram showing a fourth embodiment of the present invention.

### Best Mode for Carrying Out the Invention

Embodiments of the present invention will be described below in detail with reference to drawings.

### (First embodiment)

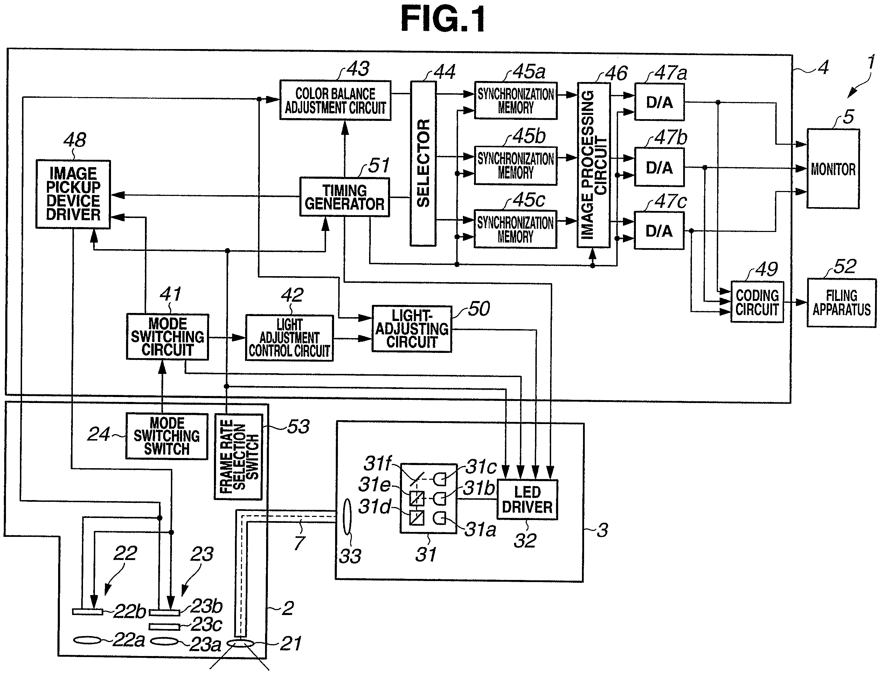

Fig. 1 is a block diagram showing a medical instrument according to a first embodiment of the present invention.

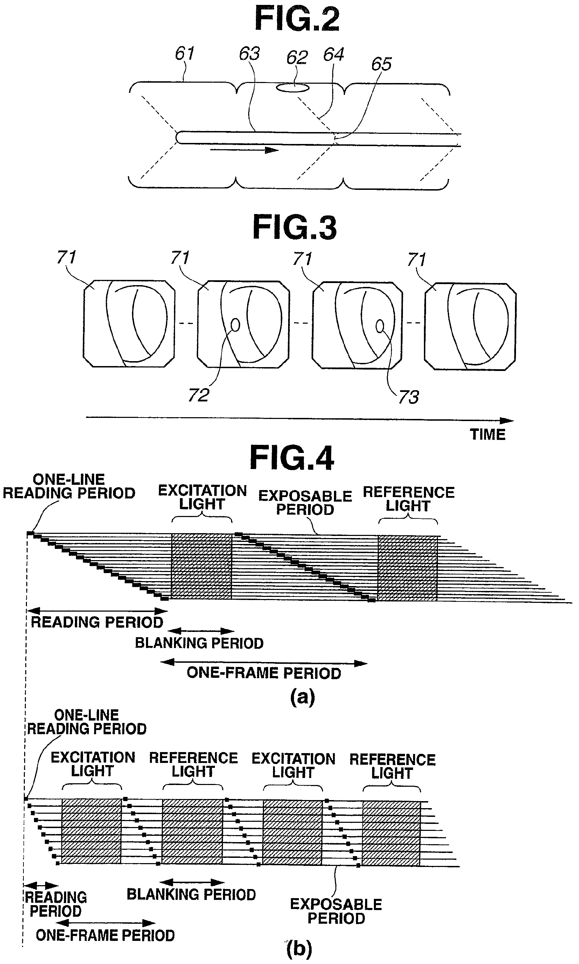

First, a technique of the present embodiment for changing a frame rate will be described with reference to Figs. 2 to 6. Fig. 2 is a diagram showing a state of fluorescence observation.

An endoscope insertion portion 63 is inserted into a lumen of a large intestine mucosa 61 or the like. The endoscope insertion portion 63 is provided with an image pickup section not shown, and an observation range of the image pickup section is shown by a broken line in Fig. 2. Fluorescence observation of the large intestine mucosa 61 in the lumen is performed while the endoscope insertion portion 63 is being moved in the direction of an arrow in Fig. 2. Note that, on a part of the large intestine mucosa 61, a marker accumulation area 62 exists where fluorescence medicine has accumulated.

Fig. 3 is a diagram showing each frame of a moving image 71 obtained by the fluorescence observation in Fig. 2.

The image pickup section provided in the endoscope insertion portion 63 picks up an image of the inside of the lumen and outputs a moving image. As shown in Fig. 3, on each frame of the moving image 71, not only the large intestine mucosa 61 but also image parts 72 and 73 corresponding to the marker accumulation area 62 are shown. When the moving speed of the endoscope insertion portion 63 is high, a period during which the marker accumulation area 62 is shown among the respective frames of the moving image 71 is relatively short (the number of frames is small). Therefore, there is a disadvantage that an observer easily misses the images 72 and 73 of the marker accumulation area 62. Note that, also in the case where the object (mucosa) moves actively due to pulsation or the like, a similar problem occurs. Not only at the time of fluorescence observation but also at the time of special-light observation such as narrowband observation, a similar problem also occurs.

Therefore, in the present embodiment, by switching the frame rate of the image pickup section provided in the endoscope insertion portion or the like according to relative velocity between the image pickup section and an object, the frame rate is increased when the relative velocity between the image pickup section and the object is high. Thereby, followability of a motion picture is improved to reduce the risk of missing.

In this case, by controlling timings of radiating excitation light and reference light for performing fluorescence observation and reading from the image pickup section, the frame rate is improved without shortening an exposure time period for detecting fluorescence, in the present embodiment. Thereby, even in the case of increasing the frame rate, visibility of a fluorescent area is improved, preventing a picked-up image from being dark.

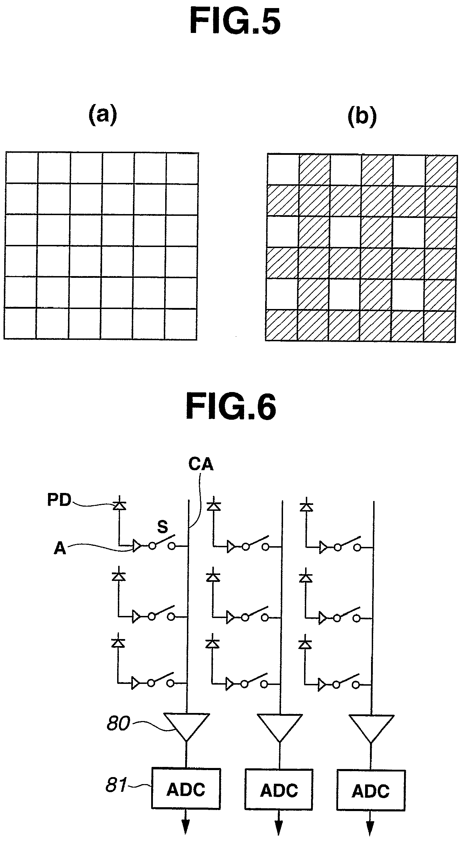

Figs. 4 and 5 are for illustrating the process in the present embodiment. Fig. 4 is a diagram for illustrating a relationship between reading of an image signal from an image pickup section and timings of radiating illuminating light, and Fig. 5 is a diagram for illustrating pixels read from the image pickup section. Figs. 4(a) and 5(a) show control performed in the case where relative velocity between the image pickup section and an object is relatively slow, and Figs. 4(b) and 5(b) show control performed in the case where the relative velocity between the image pickup section and the object is relatively fast. Note that Fig. 5 shows only vertical and horizontal 6x6 pixels of a partial area of a pixel area by a square frame.

In the present embodiment, a CMOS sensor is used as an image pickup device to pick up a fluorescence image from an object, as described later. Thinning-out reading from the CMOS sensor is performed according to the frame rate.

Fig. 6 is a schematic circuit diagram showing a configuration of the CMOS sensor. In the CMOS sensor, pixels are arranged in a matrix, and each pixel is configured with a photodiode PD, an amplification section A and a switch S. The photodiode PD generates charge according to received light. After being amplified by the amplification section A, voltage change due to the charge generated in the photodiode PD is outputted to a column line CA via the switch S.

All switches S on one column are connected to a common column line CA. By switches S on the same line being turned on at the same time, signals are provided from all pixels on the same line to respective CDS circuits 80 via the respective column lines CA. By switches S being selectively turned on for each line by a vertical scanning circuit not shown, signals of all pixels are outputted via the column lines CA.

Each CDS circuit 80 removes reset noise from the signals inputted via each column line CA and, after that, outputs the signals to each ADC 81. Each ADC 81 converts the inputted signals to digital signals and, after that, outputs the signals. Outputs from each ADC 81 are sequentially outputted by a horizontal scanning circuit not shown.

When reading of pixels of one line by the horizontal scanning circuit ends, reading of pixels of a next one line is performed. Note that, each time reading of pixels of one line ends, each pixel is reset for each line.

In Figs. 4(a) and 4(b), each horizontal line indicates a time axis, and processing for each reading line of the image pickup section is shown. One horizontal line corresponds to one reading line, and a thick line part on each horizontal line indicates a period of reading pixels of one line of the image pickup device. Note that, in Figs. 4(a) and 4(b), the number of horizontal lines differs from the actual number of lines of the image pickup device because of simplification of the drawings.

Fig. 4(a) shows that pixels of all lines of the image pickup device are read from the image pickup device. As shown in Fig. 4(a), when reading of one line indicated by a thick line ends, reading of a next line is performed. A reading period shown in Fig. 4(a) is a period required for reading of all the line, that is, reading of one screen. After the reading period ends and a predetermined blanking period elapses, reading of a next screen is performed line by line as indicated by the thick lines in Fig. 4(a).

As described above, each time reading of one line ends, pixels of the read line are reset. A period between the thick lines in Fig. 4 is an exposable period during which exposure is performed at pixels of each line. As shown in Fig. 4, the exposable period occurs at a timing different for each line.

In an endoscope, since the image pickup section is inserted into a body cavity, return light of illumination light enters the image pickup section and charge is accumulated in the pixels only during a period during which the illumination light is radiated to an object. Therefore, the period during which illumination light is radiated, within the exposable period, is an actual exposure period.

In fluorescence observation, not only excitation light for generating fluorescence but also reference light for acquiring the shape of an insertion site may be radiated to an object. By radiating excitation light and reference light in time sharing and integrating a fluorescence image obtained by radiating the excitation light and a reference light image obtained by radiating the reference light, a composite observed image which makes it possible to observe the shape of the insertion site and a marker accumulation area at the same time can be produced. For example, by switching between the excitation light and the reference light for each one-frame period, the composite observed image is obtained.

The reading period indicated by the thick line in Fig. 4(a) is an exposable period of a previous frame for a line before being read and is an exposable period of a following frame for a line after being read. Therefore, if excitation light or reference light is radiated during reading, exposure covering two successive frames is performed, and the excitation light and the reference light are mixed. Consequently, a fluorescence image and a reference light image cannot be obtained.

Because of this reason, in the present embodiment, an excitation light radiation period and a reference light radiation period are set in a blanking period between reading periods as shown in Fig. 4(a). In order to increase the frame rate, a method of shortening the blanking period is conceivable, in consideration of limitation of speed-up of reading. However, in the case of performing radiation, switching among multiple kinds of illumination light, such as excitation light and reference light, for each screen, it is necessary to radiate the illumination light during a blanking period. Therefore, an illumination period, that is, an exposure period is also shortened by shortening the blanking period, and an image from the image pickup device is dark. Especially, since fluorescence corresponding to excitation light is relatively dark, there is a possibility that the visibility of a marker accumulation area significantly deteriorates due to shortening of the exposure period.

Accordingly, in the present embodiment, pixels are read being thinned out so as to increase the frame rate without shortening the exposure period. In Figs. 5(a) and 5(b), pixels which are not read are indicated by shaded parts. Fig. 5(a) corresponds to Fig. 4(a) and shows that all pixels are read. In comparison, in a high-speed frame rate mode, pixels are read being thinned out, as shown in Fig. 5(b). For example, in the example in Fig. 5(b), pixels are read for each column and for each line. Therefore, the number of pixels read for one line corresponds to 1/2 in the case of all-pixel reading, and a time period required for reading one line corresponds to about 1/2 of a time period for reading all pixels of one line. Furthermore, since pixels of the number of lines corresponding to 1/2 of the number of lines of one screen are read, it is possible to perform reading of one screen in a time period corresponding to about 1/2 of a time period required for reading all lines of one screen.

Fig. 4(b) shows an example of such thinning-out reading, and a one-screen reading period corresponds to about 1/4 of the one-screen reading period in Fig. 4(a). In comparison, in the present embodiment, excitation light and reference light illumination periods are similar to those in Fig. 4(a), as shown in Fig. 4(b). Even in this case, it is also possible to increase the frame rate because the one-screen reading period is shortened.

By performing the reading control and illumination timing control corresponding to the reading control shown in Fig. 4(b), it is possible to increase the frame rate without shortening the exposure period. Note that the examples in Figs. 4 and 5 show an example of thinning out pixels by 1/2 in horizontal and vertical directions. However, the thinning-out interval and the like can be set appropriately.

### (Circuit configuration)

In Fig. 1, an endoscope 2 is configured to have an illumination optical system 21 that emits light supplied from a light source device 3 and transmitted by a light guide 7 to an object; an image pickup section for white color light observation 22, an image pickup section for fluorescence observation 23, a mode switching switch 24 capable of performing an operation for switching the observation mode of an endoscope apparatus 1, and a frame rate selection switch 53 for performing switching of the frame rate.

The image pickup section for white color light observation 22 is configured to have an objective optical system 22a that forms an image of an object, a CMOS sensor 22b in which an image pickup surface provided with a primary color filter is arranged to correspond to the image forming position of the objective optical system 22a.

The CMOS sensor 22b is drive-controlled by an image pickup device driver 48 in a processor 4, and the CMOS sensor 22b generates an image pickup signal by performing photoelectrical conversion of return light from an object the image of which is formed on the image pickup surface, and outputs the signal to the processor 4.

The image pickup section for fluorescence observation 23 is configured to have an objective optical system 23a that forms an image of an object, a monochrome CMOS sensor 23b, the image pickup surface of which is arranged to correspond to the image forming position of the objective optical system 23a, and an excitation light cut filter 23c arranged at a pre-stage of the CMOS sensor 23b.

The CMOS sensor 23b is drive-controlled by the image pickup device driver 48 in the processor 4, and the CMOS sensor 23b generates an image pickup signal by performing photoelectrical conversion of return light from an object the image of which is formed on the image pickup surface and outputs the signal to the processor 4. The CMOS sensor 23b has a function of selecting and reading a pixel, and is configured so that which pixel in a screen is to be read is controlled by the image pickup device driver 48.

The excitation light cut filter 23c is formed being provided with such an optical characteristic that blocks the wavelength band of excitation light emitted from an excitation light LED 31b to be described later and causes each of the wavelength band of fluorescence emitted from a fluorescent substance, such as fluorescence medicine, excited by the excitation light and the wavelength band of reference light emitted from a reference light LED 31 c to be described later to be transmitted.

The mode switching switch 24 outputs an operation signal corresponding to an operation by a surgeon, to a mode switching circuit 41. The surgeon can specify a white color light observation mode or a fluorescence observation mode by the mode switching switch.

In the present embodiment, the endoscope 2 is provided with the frame rate selection switch 53, for example, at an operation section. The frame rate selection switch 53 is adapted to output a frame rate selection signal for selecting a frame rate according to an operation by the surgeon.

Note that, though the example is shown in which the frame rate selection switch 53 is provided on the endoscope 2, the frame rate selection switch 53 may be provided on a front panel, a keyboard or the like not shown of the processor 4.

The light source device 3 has an LED light source section 31, an LED driver 32, and a condensing optical system 33 that condenses light emitted by the LED light source section 31 and supplies the light to the light guide 7. The LED light source section 31 is configured to have a white color light LED 31 a, the excitation light LED 3 1 b, the reference light LED 31c, a half mirror 31d, a half mirror 31e and a mirror 31f. The white color light LED 31a is configured so as to be able to emit white color light that includes each of the wavelength bands of R (red), G (green) and B (blue) (for example, visible light of 400 to 690 nm).

The excitation light LED 31b is configured so as to be able to emit excitation light with a wavelength band capable of exciting a fluorescent substance such as fluorescence medicine (for example, near infrared light of 700 nm). The reference light LED 31c is configured so as to be able to emit reference light with a wavelength band that overlaps neither the wavelength band of excitation light emitted from the excitation light LED 31b nor the wavelength band of fluorescence emitted from a fluorescent substance, such as fluorescence medicine, excited by the excitation light (for example, near infrared light of 800 nm).

The half mirror 31d is arranged on an optical path between the white color light LED 31a and the condensing optical system 33, and configured being provided with such an optical characteristic that causes white color light emitted from the white color light LED 31a to be transmitted to the condensing optical system 33 side and reflects excitation light and reference light emitted via the half mirror 31e to the condensing optical system 33 side.

The half mirror 31e is arranged on an optical path between the half mirror 31d and the mirror 31f, and is configured being provided with such an optical characteristic that reflects excitation light emitted from the excitation light LED 31b to the half mirror 31d side and causes reference light emitted via the mirror 31f to be transmitted to the half mirror 31 d side. The mirror 31 f is configured being provided with such an optical characteristic that reflects reference light emitted from the reference light LED 31 c to the half mirror 31e side.

The LED driver 32 is configured to be able to supply a drive current for driving each LED provided in the LED light source section 31. Therefore, for example, as the size of the drive current supplied from the LED driver 32 to the LED light source section 31 changes, the strength of light (white color light, excitation light and reference light) emitted from the LED light source section 31 changes. Otherwise, a drive current during an illumination period may be supplied in a pulse waveform to change the strength of light by adjusting the pulse width.

On the other hand, the LED driver 32 operates so as to cause each LED provided in the LED light source section 31 to emit light or stop light emission according to control by the processor 4. That is, the LED driver 32 controls the LED light source section 31 according to the observation mode. The observation mode can be set by the mode switching switch 24.

The mode switching circuit 41 generates a mode switching control signal for causing each section of the endoscope apparatus 1 to perform an operation corresponding to an observation mode selected by an operation of the mode switching switch 24, and outputs the signal to a light adjustment control circuit 42, the LED driver 32 and the image pickup device driver 48.

The light adjustment control circuit 42 is adapted to set a brightness target value and the like corresponding to an observation mode, for a light-adjusting circuit 50. The brightness target value corresponding to an observation mode is set for the light-adjusting circuit 50 by the light adjustment control circuit 42, and the light-adjusting circuit 50 generates a light adjustment signal for adjusting brightness of an image pickup signal on the basis of the set target value and the level of the image pickup signal and outputs the signal to the LED driver 32.

The LED driver 32 is also given a timing signal from a timing generator 51. The timing generator 51 generates and outputs a timing signal for causing operations of the respective sections of the processor 4 to be appropriately synchronized. For example, when the endoscope apparatus 1 is switched to the fluorescence observation mode, the timing generator 51 generates and outputs a timing signal for causing each section of the processor 4 to perform an operation synchronized with a period during which the excitation light LED 31b emits light (or stops light emission) and a period during which the reference light LED 3 c emits light (or stops light emission).

When detecting that the endoscope apparatus 1 has been switched to the white color light observation mode, on the basis of a mode switching control signal outputted from the mode switching circuit 41 of the processor 4, the LED driver 32 operates so as to cause the white color light LED 31a to emit light and cause the excitation light LED 31b and the reference light LED 31c to stop light emission. When detecting that the endoscope apparatus 1 has been switched to the fluorescence observation mode, on the basis of a mode switching control signal outputted from the mode switching circuit 41, the LED driver 32 operates so as to cause the white color light LED 31a to stop light emission and cause the excitation light LED 31b and the reference light LED 31 c to alternately emit light.

The processor 4 has a color balance adjustment circuit 43 that performs color balance adjustment processing, a multiplexer 44 that performs an operation related to sorting of signals, synchronization memories 45a, 45b and 45c, an image processing circuit 46 that performs predetermined image processing, and DACs 47a, 47b and 47c that perform D/A conversion processing.

Both of an image pickup signal outputted from the CMOS sensor 22b in the white color light observation mode and a digital image pickup signal outputted from the CMOS sensor 23b in the fluorescence observation mode are inputted to the processor 4. The color balance adjustment circuit 43 performs color balance adjustment processing of a digital image pickup signal on the basis of a timing signal from the timing generator 51 and outputs the signal to a selector 44.

The selector 44 separates the image pickup signal outputted from the color balance adjustment circuit 43 on the basis of a timing signal from the timing generator 51 into signals of three channels according to the mode and outputs the respective separated signals, allocating the signals to the synchronization memories 45a, 45b and 45c. Each of the synchronization memories 45a, 45b and 45c has a configuration capable of temporarily storing the respective signals outputted from the selector 44. For example, at the time of the white color light observation mode, signals of respective color components separated from an image pickup signal are stored in the synchronization memories 45a, 45b and 45c, respectively. At the time of the fluorescence observation mode, signals of a fluorescence image obtained by excitation light exposure and a reference image obtained by reference light exposure are stored in the synchronization memories 45a, 45b and 45c.

The image processing circuit 46 reads the signals of the respective channels stored in the synchronization memories 45a, 45b and 45c at the same time on the basis of a timing signal from the timing generator 51 and, after that, performs image processing, such as gamma correction, for each of the read signals. Then, the image processing circuit 46 assigns the signals which have received image processing, such as gamma correction, to a first color channel (for example, an R component), a second color channel (for example, a G component) and a third color channel (for example, a B component), respectively, to output the signals to the DACs 47a, 47b and 47c.

The signals of the first to third color channels outputted from the image processing circuit 46 are converted to analog signals at the DACs 47a, 47b and 47c, respectively, and outputted to a monitor 5. Thereby, an observed image corresponding to each observation mode is displayed on the monitor 5.

Note that outputs of the DACs 47a, 47b and 47c are also given to a coding circuit 49. The coding circuit 49 performs coding processing of the inputted signals and outputs the signals to a filing apparatus 52. The filing apparatus 52 performs filing of the coded data which have been inputted.

The image pickup device driver 48 is provided with a timing signal from the timing generator 51 and drives the CMOS sensor 22b and the CMOS sensor 23b. For example, when detecting that the endoscope apparatus 1 has been switched to the white color light observation mode, on the basis of a mode switching control signal of the mode switching circuit 41, the image pickup device driver 48 drives the CMOS sensor 22b and stops driving of the CMOS sensor 23b. Furthermore, when detecting that the endoscope apparatus 1 has been switched to the fluorescence observation mode, on the basis of a mode switching control signal, the image pickup device driver 48 drives the CMOS sensor 23b and stops driving of the CMOS sensor 22b.

In the present embodiment, the LED driver 32, the timing generator 51 and the image pickup device driver 48 are adapted to be controlled by a frame rate selection signal from the frame rate selection switch 53. For example, it is assumed that a normal frame rate mode and a high-speed frame rate mode can be specified by a frame rate selection signal. In the normal frame rate mode, for example, the reading and illumination control shown in Fig. 4(a) is performed.

That is, in this case, the timing generator 51 generates a timing signal corresponding to the number of horizontal and vertical pixels of the CMOS sensor 23b, and generates various timing signals for driving the horizontal and vertical scanning circuits, for example, a horizontal synchronization signal, a vertical synchronization signal and a blanking signal on the basis of the timing signal. The image pickup device driver 48 drives the CMOS sensor 23b using the various timing signals from the timing generator 51 to read all lines of the CMOS sensor 23b one by one. When reading of each line ends, the image pickup device driver 48 causes pixels of each line to be into an exposable state after resetting the pixels. When reading of the pixels of all the lines of the CMOS sensor 23b ends, the image pickup device driver 48 performs reading of a next screen after a blanking period based on the blanking signal.

The timing generator 51 also outputs a blanking signal to the LED driver 32. The LED driver 32 controls radiation on the basis of a mode switching control signal and a frame rate selection signal. The LED driver 32 is adapted to, in the fluorescence observation mode, alternately radiate excitation light and reference light within a blanking period shown by the blanking signal. Thereby, the LED driver 32 alternately radiates excitation light and reference light at timings shown in Fig. 4(a) when the normal frame rate mode is specified at the time of the fluorescence observation mode.

On the other hand, when the high-speed frame rate mode is specified, for example, reading and illumination control shown in Fig. 4(b) is performed. That is, in this case, the timing generator 51 generates a timing signal corresponding to the number of horizontal and vertical pixels of the CMOS sensor 23b, and generates various timing signals for driving the horizontal and vertical scanning circuits at a high speed, for example, a horizontal synchronization signal, a vertical synchronization signal and a blanking signal on the basis of the timing signal.

The image pickup device driver 48 drives the CMOS sensor 23b using the various timing signals from the timing generator 51 to perform thinning-out reading from the CMOS sensor 23b. For example, the image pickup device driver 48 performs thinning-out reading for each column and performs thinning-out reading for each line, from the CMOS sensor 23b. That is, in this case, a horizontal cycle is reduced to about 1/2, and a vertical cycle is also reduced to about 1/2, in comparison with the case of all-pixel reading. Therefore, the one-screen reading period in this case corresponds to about 1/4 at the time of all-pixel reading.

When the thinning-out reading of reading lines ends, the image pickup device driver 48 resets pixels of the reading lines. Note that the image pickup device driver 48 may reset pixels of thinned-out lines. When reading of the pixels of the reading lines of the CMOS sensor 23b ends, the image pickup device driver 48 performs reading of a next screen after a blanking period based on the blanking signal.

In the present embodiment, the timing generator 51 sets a sufficient blanking period in both of the normal frame rate mode and the high-speed frame rate mode. For example, the timing generator 51 sets the same blanking period for the normal frame rate mode and the high-speed frame rate mode. Thereby, in the fluorescence observation mode also, the LED driver 32 alternately radiates excitation light and reference light during a sufficient blanking period. Thus, the LED driver 32 alternately radiates excitation light and reference light, for example, at timings shown in Fig. 4(b) when the high-speed frame rate mode is specified at the time of the fluorescence observation mode.

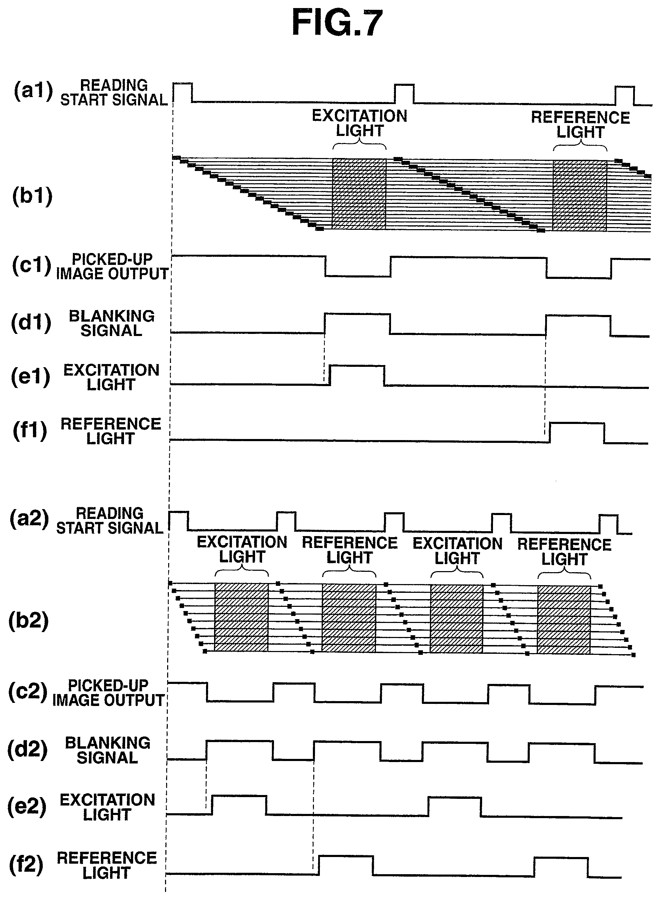

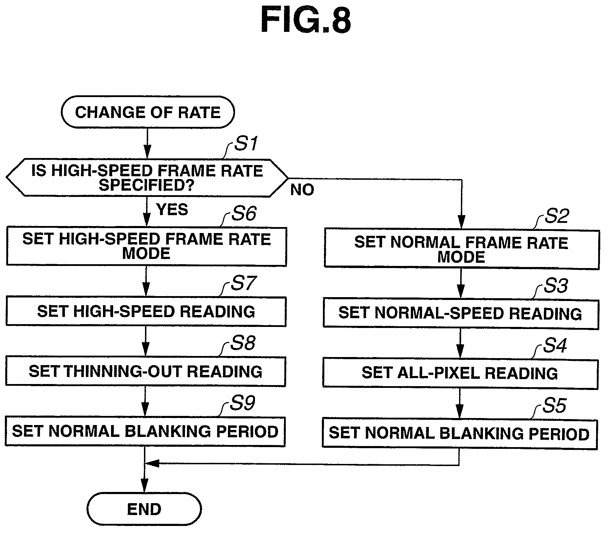

Next, an operation of the embodiment configured as described above will be described with reference to a timing chart in Fig. 7 and a flowchart in Fig. 8. Figs. 7(a1) to 7(f1) show control in the normal frame rate mode, and Figs. 7(a2) to 7(f2) show control in the high-speed frame rate mode. Figs. 7(a1) and 7(a2) show reading start signals; Figs. 7(b1) and 7(b2) correspond to Figs. 4(a) and 4(b); Figs. 7(c1) and 7(c2) show picked-up image outputs; Figs. 7(d1) and 7(d2) show blanking signals; Figs. 7(e1) and 7(e2) show excitation light radiation timings; and Figs. 7(f1) and 7(f2) show reference light radiation timings.

The surgeon specifies the white color light observation mode or the fluorescence observation mode by operating the mode switching switch 24. The mode switching circuit 41 generates a mode switching control signal based on the operation of the mode switching switch 24 and outputs the generated mode switching control signal to the light adjustment control circuit 42, the LED driver 32 and the image pickup device driver 48.

For example, it is assumed that the white color light observation mode is specified by the surgeon now. In this case, the LED driver 32 causes the white color light LED 31 a to emit light and causes the excitation light LED 31b and the reference light LED 31c to stop light emission, on the basis of a mode switching control signal. The image pickup device driver 48 drives the CMOS sensor 22b and stops driving of the CMOS sensor 23b, on the basis of the mode switching control signal.

Thereby, white color light supplied from the light source device 3 is emitted to an object via the light guide 7 and the illumination optical system 21, and return light (reflected light) of the white color light forms an image on the image pickup surface of the CMOS sensor 22b. Then, an image pickup signal obtained by performing image pickup of the return light (reflected light) of the white color light is outputted from the CMOS sensor 22b.

The image pickup signal outputted from the CMOS sensor 22b is inputted to the light-adjusting circuit 50. The light-adjusting circuit 50 generates a light adjustment signal for adjusting the brightness of the image pickup signal to a brightness target value in the white color light observation mode on the basis of the mode switching control signal and outputs the light adjustment signal to the LED driver 32. The LED driver 32 causes the brightness of the image pickup signal to correspond to the target value by changing a driving current to be supplied to the white color light LED 31a on the basis of the light adjustment signal.

On the other hand, the image pickup signal outputted from the CMOS sensor 22b is outputted to the monitor 5 as a video signal after passing through each of the sections of the color balance adjustment circuit 43, the selector 44, the synchronization memories 45a to 45c, the image processing circuit 46 and the DACs 47a to 47c. In this way, an observed image (a color image) corresponding to the white color light observation mode is displayed on the monitor 5.

It is assumed that the surgeon performs fluorescence observation of a subject next. Before observing a desired observation site inside the subject, the surgeon gives fluorescence medicine to be accumulated in lesion tissue of the desired observation site, to the subject. After that, the surgeon positions the distal end portion of the endoscope 2 near the desired observation site in the subject by performing an operation of inserting the endoscope 2, seeing an observed image displayed on the monitor 5. Then, in such a state, the surgeon selects the fluorescence observation mode by operating the mode switching switch 24.

When detecting that the fluorescence observation mode has been selected by the operation of the mode switching switch 24, the mode switching circuit 41 generates a mode switching control signal corresponding to the fluorescence observation mode and outputs the signal to the light adjustment control circuit 42, the LED driver 32 and the image pickup device driver 48.

On the basis of the mode switching control signal, the LED driver 32 causes the white color light LED 31 a to stop light emission, and causes the excitation light LED 31b to emit light or causes the excitation light LED 31b and the reference light LED 31c to alternately emit light. Note that the LED driver 32 controls periods during which the excitation light LED 31b is caused to emit light and stop light emission and periods during which the reference light LED 31c is caused to emit light and stop light emission, on the basis of a timing signal from the timing generator 51. The image pickup device driver 48 drives the CMOS sensor 23b and stops driving of the CMOS sensor 22b, on the basis of the mode switching control signal.

It is assumed that the normal frame rate mode is specified now. In this case, the timing generator 51, the image pickup device driver 48 and the LED driver 32 advance the process from step S1 to step S2 in Fig. 8 to set the normal frame rate mode at steps S2 to S5, on the basis of a frame rate selection signal from the frame rate selection switch 53. That is, generation of various timing signals required for the normal frame rate mode, setting of all-pixel reading and setting of a normal blanking period are performed.

That is, the timing generator 51 generates a blanking signal shown in Fig. 7(d1). The blanking signal is set for periods among periods of reading respective screens of the CMOS sensor 23b.

The blanking signal is provided to the LED driver 32, and the LED driver 32 causes excitation light or reference light to be emitted from the LED light source section 31 after a predetermined delay time period after a start of a blanking period shown by the blanking signal, before an end of the blanking period (Figs. 7(e1), 7(f1) and shaded parts of Fig. 7(b1)). Return light from an object based on radiation of the excitation light and the reference light forms an image on the CMOS sensor 23b as an optical image, and charge is accumulated in each pixel of the CMOS sensor 23b. In this way, a picked-up image based on excitation light or reference light is obtained during a blanking period within an exposable period of the CMOS sensor 23b.

The image pickup device driver 48 generates a reading start signal (Fig. 7(a1)) for specifying a reading start timing of the CMOS sensor 23b on the basis of various timing signals from the timing generator 51 and transmits the reading start signal to the CMOS sensor 23b. The CMOS sensor 23b starts reading in synchronization with a rising timing of the reading start signal. Note that, in this case, the image pickup device driver 48 specifies all-pixel reading to the CMOS sensor 23b. In this way, signals based on charge accumulated during an exposure period based on illumination of excitation light or reference light are sequentially read from the CMOS sensor 23b line by line during a high level period in Fig. 7(c1). A fluorescence image and a reference light image read from the CMOS sensor 23b are provided to and processed by the processor 4, and a composite observed image is displayed on the monitor 5.

Here, it is assumed that, since relative velocity between the endoscope 2 and an object is relatively high, an operator operates the frame rate selection switch 53 to specify the high-speed frame rate mode. In this case, the timing generator 51, the image pickup device driver 48 and the LED driver 32 advance the process from step S1 to step S6 to set the high-speed frame rate mode at steps S6 to S9, on the basis of a frame rate selection signal.

That is, the timing generator 51 generates a timing signal with a high frequency corresponding to the high-speed frame rate mode on the basis of the frame rate selection signal. The image pickup device driver 48 performs thinning-out reading from the CMOS sensor 23b on the basis of the frame rate selection signal.

The timing generator 51 outputs a blanking signal shown in Fig. 7(d2) at the time of the high-speed frame rate mode. As shown in Fig. 7(d2), the cycle of the blanking signal at the time of the high-speed frame rate mode is short in comparison with that at the time of the normal frame rate mode, but the blanking period does not change. The LED driver 32 causes excitation light or reference light to be emitted from the LED light source section 31 (Figs. 7(e2), 7(f2), and shaded parts in Fig. 7(b2)) after a predetermined delay time period after a start of a blanking period shown by the blanking signal, before an end of the blanking period. Return light from an object based on radiation of the excitation light and the reference light forms an image on the CMOS sensor 23b as an optical image, and charge is accumulated in each pixel of the CMOS sensor 23b. In this way, a picked-up image based on excitation light or reference light is obtained during a blanking period in an exposable period of the CMOS sensor 23b.

On the other hand, the image pickup device driver 48 generates a reading start signal (Fig. 7(a2)) for specifying a reading start timing of the CMOS sensor 23b on the basis of various timing signals from the timing generator 51 and transmits the reading start signal to the CMOS sensor 23b. The CMOS sensor 23b starts reading in synchronization with a rising timing of the reading start signal. In this case, the image pickup device driver 48 specifies thinning-out reading to the CMOS sensor 23b.

For example, the image pickup device driver 48 specifies reading every other column and every other line to the CMOS sensor 23b. The CMOS sensor 23b outputs a signal based on accumulated charge every other column and every other line during a high level period in Fig. 7(c2). A fluorescence image and a reference light image read from the CMOS sensor 23b are provided to the processor 4 and outputted to the monitor 5. In this case, the one-screen reading period is reduced to 1/4 in comparison with a reading period at the time of all-pixel reading.

In this way, reading at a high frame rate shown in Figs. 7(a2) to 7(f2) is performed. At the time of the high-speed frame rate mode, a radiation time period of excitation light and reference light is the same as that at the time of the normal frame rate mode, and it does not happen that a picked-up image is dark.

As described above, in the present embodiment, reading at a high frame rate is performed when relative velocity between the image pickup section and an object is relatively high. Thereby, the number of picked-up images of a marker accumulation area increases in a picked-up moving image, and the risk of missing the marker accumulation area can be reduced. At the time of the high-speed frame rate mode, the rate is increased by shortening a reading period by performing thinning-out reading, without shortening a blanking period. Even in the high-speed frame rate mode, a sufficient blanking period is ensured to lengthen a time period of radiating excitation light, reference light and the like, so that a sufficiently bright image can be obtained.

Note that, though operations in two kinds of frame rates, the normal frame rate mode and the high-speed frame rate mode, have been described in the above embodiment, it is apparent that, by setting three or more kinds of frame rates, an operation can be performed in a desired frame rate among the three kinds of frame rates, by an operator's operation.

Though an example of performing reading at a high frame rate at the time of fluorescence observation has been described in the above embodiment, it is apparent that, in special-light observation, such as narrow band light observation, reading at a high frame rate may be also performed with the use of the technique of the above embodiment if relative velocity between an image pickup section and an object is relatively high.

Though an example of performing radiation within a blanking period in the case of radiating two kinds of illumination light, excitation light and reference light, has been described in the above embodiment, radiation may be performed within a blanking period in the case of radiating one kind of illumination light also, such as radiating only excitation light to perform fluorescence observation. In a CMOS sensor, distortion due to difference among exposure periods for lines is caused by a rolling shutter in a picked-up image of an object with motion. In comparison, by not performing radiation during a reading period as in the present embodiment, an advantage is obtained that influence by the distortion based on the rolling shutter can be eliminated.

In the above embodiment, a form of improving reading speed per frame by a reading pixel thinning-out process is shown on the assumption that a time period for reading one frame is restricted by the reading speed of a CMOS sensor. In an actual endoscope, however, it is also assumed that the time period for reading one frame is restricted not only by the reading speed of the CMOS sensor but also by signal transmission speed at the time of transmitting a video signal after reading to a processor. Therefore, in addition to the reading pixel thinning-out process in the above embodiment, so-called binning reading may be performed in which, at the time of reading of the CMOS sensor, addition processing of signals of four pixels is performed to read the signals as a signal of one pixel. Since the amount of information of image signals transmitted to the processor is reduced by binning reading, it is possible to shorten the reading time period per frame without being restricted by the transmission speed.

### (Second embodiment)

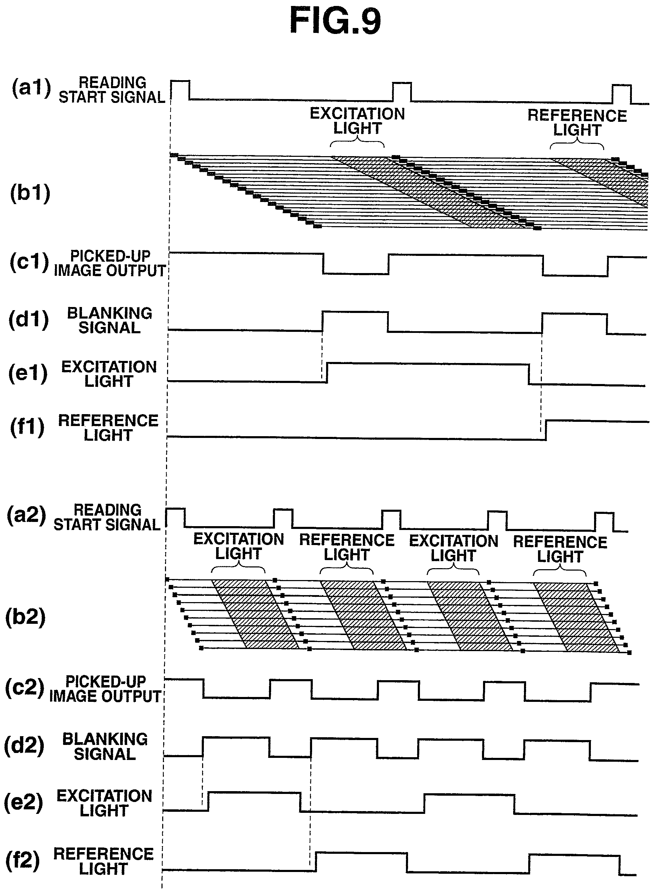

Fig. 9 is a timing chart for illustrating a second embodiment not forming part of the present invention. Figs. 9(a1) to 9(f1) and Figs. 9(a2) to 9(f2) correspond to Figs. 7(a2) to 7(f2), respectively.

A hardware configuration of the present embodiment is similar to that of the first embodiment, and the present embodiment is different from the first embodiment only in illumination control of the LED driver 32 and reading control of the image pickup device driver 48.

In the present embodiment, the LED driver 32 causes excitation light or reference light to be emitted from the LED light source section 31 during a period after a predetermined delay time period after an end of a one-screen reading period, before a predetermined time period before an end of a next one-screen reading period, as shown in Figs. 9(a1) to 9(f1) and Figs. 9(a2) to 9(f2). That is, excitation light and illumination light are radiated to an object during almost the whole exposable period of each line of the CMOS sensor 23b.

On the other hand, the image pickup device driver 48 resets pixels of each line after reading of each line ends. After the reset, exposure is performed on the pixels of each line. Therefore, in the present embodiment, the image pickup device driver 48 performs control so as to discard charge accumulated for each line once using an electronic shutter function and sequentially start exposure for each line again, after a predetermined delay time period after an end of reading of one screen.

Thereby, exposure of fluorescence based on excitation light and exposure of reference light are performed during periods shown indicated by shaded parts in Figs. 9(b1) and 9(b2).

In the embodiment configured as above, the image pickup device driver 48 is given various timing signals from the timing generator and transmits a reading start signal (Figs. 7(b1) and 7(b2) to the CMOS sensor 23b. The CMOS sensor 23b starts reading in synchronization with the reading start signal and sequentially outputs image pickup data line by line.

On the other hand, the LED driver 32 causes emission of excitation light or reference light to be started after a predetermined delay time period after a start of a blanking period on the basis of a blanking signal, and causes the emission to be continued until the time point of an end of next one-screen reading.

The image pickup device driver 48 resets image-pickup charge of the CMOS sensor 23b at an end of reading of each line. Excitation light or reference light is being radiated after the reset of each line, and exposure is started at each pixel immediately after the reset. The image pickup device driver 48 once discards charge temporarily accumulated so far using the electronic shutter function after the end of a one-screen reading period, and performs control to start exposure sequentially for each line again.

Thereby, as shown in Figs. 9(b1) and 9(b2), an effective exposure period for each line is equal to a blanking period. In the high-speed frame rate mode in Figs. 9(a2) to 9(f2), the one-screen reading period is shortened, and the frame rate is increased, in comparison with the normal frame rate mode in Figs. 9(a1) to 9(f1). In comparison, the exposure period is the same between the normal frame rate mode and the high-speed frame rate mode.

Thus, in the present embodiment also, an advantage similar to the first embodiment can be obtained. Note that, in the present embodiment, a period of light radiation from the LED light source section 31 is similar to that at the time of the white color light observation mode, and illumination control is easy.

### (Third embodiment)

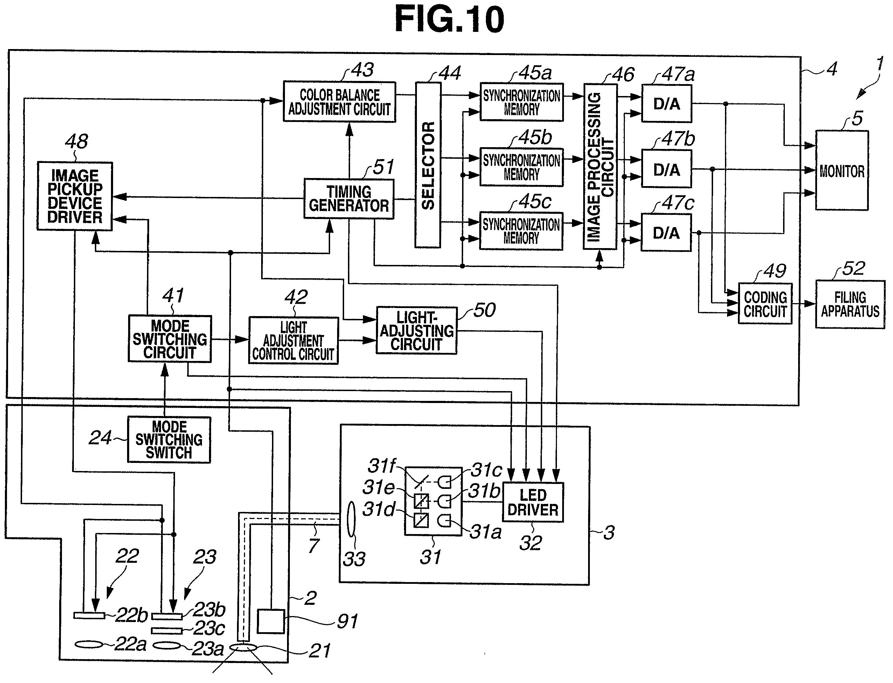

Fig. 10 is a block diagram showing a third embodiment not forming part of the present invention. In Fig. 10, the same components as in Fig. 1 are given the same reference numerals, and description thereof will be omitted.

The present embodiment is different from each of the above embodiments in that a motion detection section 91 is provided instead of the frame rate selection switch 53. In each of the above embodiments, any frame rate among multiple frame rates is selected by an operator operating the frame rate selection switch 53. In comparison, in the present embodiment, motion of the image pickup section is detected, and any frame rate among multiple frame rates is selected on the basis of the detected motion.

The motion detection section 91 for detecting motion of the image pickup section for white color light observation 22 and the image pickup section for fluorescence observation 23 is arranged near the image pickup section for fluorescence observation 23 at the distal end of the endoscope 2. The motion detection section 91 is configured, for example, with an acceleration sensor or the like, and outputs a frame rate selection signal for selecting a frame rate according to motion of the image pickup sections 22 and 23. As the motion of the image pickup sections 22 and 23 is faster, the motion detection section 91 outputs a frame rate selection signal for selecting a higher frame rate.

The motion detection section 91 may, for example, detect acceleration of the image pickup sections 22 and 23, determine average acceleration within a predetermined time period, and determine a frame rate to be selected on the basis of which range the average acceleration is included in. For example, the motion detection section 91 may output a frame rate selection signal for specifying the normal frame rate mode if the average acceleration is equal to or below a predetermined threshold and output a frame rate selection signal for specifying the high-speed frame rate mode when the average acceleration exceeds the predetermined threshold.

In the embodiment configured as described above, motion of the image pickup sections 22 and 23 is detected by the motion detection section 91. As the motion of the image pickup sections 22 and 23 is faster, the motion detection section 91 outputs a frame rate selection signal for selecting a higher frame rate.

The frame rate selection signal from the motion detection section 91 is given to the timing generator 51, the image pickup device driver 48 and the LED driver 32. The subsequent operation is similar to that in each of the above embodiments. A reading period based on the frame rate selection signal is set, while an exposure period of excitation light and reference light is set almost constant irrespective of a frame rate.

Thereby, in the present embodiment also, it is possible to, by performing reading at a high frame rate while ensuring sufficient brightness, reduce the risk of missing an observation site.

The present embodiment is excellent in convenience because switching among frame rates is automatically performed.

### (Fourth embodiment)

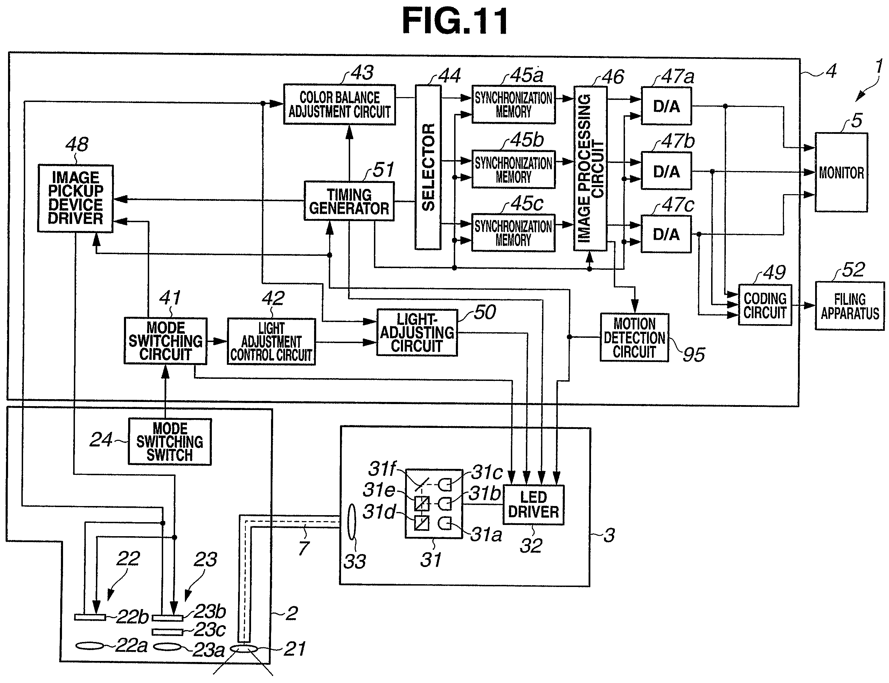

Fig. 11 is a block diagram showing a fourth not forming part of the present invention. In Fig. 11, the same components as in Fig. 10 are given the same reference numerals, and description thereof will be omitted.

The present embodiment is different from the third embodiment in that a motion detection circuit 95 is provided instead of the motion detection section 91. The motion detection circuit 95 is provided in the processor 4. An image signal is provided from the image processing circuit 46 to the motion detection circuit 95. The motion detection circuit 95 detects relative motion between the image pickup section and an object from a calculation result of two successive frames of a time-sequentially inputted image signal.

For example, the motion detection circuit 95 extracts a feature point of an object in an image, such as an edge part, and estimates relative velocity between the image pickup section and the object from the amount of movement of the feature point in the two successive frames. The motion detection circuit 95 determines which range the relative amount of movement is included in, and sets a frame rate according to the determined range. That is, as the relative amount of movement is larger, the motion detection circuit 95 determines a higher frame rate. The motion detection circuit 95 outputs a frame rate selection signal showing the determined frame rate to the timing generator 51, the image pickup device driver 48 and the LED driver 32.

Note that the motion detection circuit 95 may output a frame rate selection signal for specifying the normal frame rate mode if the relative amount of movement of a feature point between two successive frames is equal to or below a predetermined threshold and output a frame rate selection signal for specifying the high-speed frame rate mode when the relative amount of movement exceeds the predetermined threshold.

Other components, operation and an advantage are similar to those of the third embodiment. Note that, in the present embodiment, relative motion between the image pickup section and an object is detected, and it is possible to set an effective frame rate, even in the case where both of the image pickup section and the object are moving.

Note that, though an example has been described in which motion of the image pickup section and the like is detected by the motion detection section 91 or the motion detection circuit 95, and the frame rate is automatically changed according to the motion, in the third and fourth embodiments, a motion detection result may be displayed on the monitor, and the frame rate may be changed according to an operator's operation as in the first and second embodiments.

The present application is filed claiming the priority of Japanese Patent Application No. 2011-137752 filed in Japan on June 21, 2011.

## Claims

1. An endoscope apparatus (1), comprising:
an illumination section (21) which alternately irradiates a subject with special light and reference light for a predetermined blanking period,
a CMOS image pickup device (22b, 23b) which includes a plurality of lines of pixels that receive light from the subject which is irradiated with the special light from the illumination section (21) for the predetermined blanking period and generates an image pickup signal, sequentially reads the image pickup signal from the lines of pixels, during a reading period, and, after a subsequent exposure of all of the pixels with reference light for the predetermined blanking period sequentially reads the image pickup signal from the plurality of lines of pixels during a further reading period;
a frame rate selection switch (53) which sets a first frame rate and a second frame rate higher than the first frame rate of the CMOS image pickup device (22b, 23b); and
an image pickup device driver (48) which, when the second frame rate is set by the frame rate selection switch (53), performs reading pixel thinning-out to shorten the reading periods of the image pickup signal by selecting and reading predetermined pixels from selected lines of the plurality of pixels to be read from the CMOS image pickup device (22b, 23b) during the reading period,
**characterized in that**
the image pickup device driver (48), when the second frame rate is set by the frame rate selection switch (53), sets the predetermined blanking period that is the same as for the first frame rate, without shortening the predetermined blanking period.

2. The endoscope apparatus (1) according to claim 1, wherein, when reading a line of the image pickup signal from the image pickup device (22b, 23b) ends, the image pickup device driver (48) resets pixels of the read line.

3. The endoscope apparatus (1) according to claim 1, wherein
the CMOS image pickup device (22b, 23b) is a CMOS sensor in which pixels are arranged in a matrix and the pixels are arranged in respective lines in the matrix, and
the image pickup device driver (48) performs reading pixel thinning-out for each column and for each line of the CMOS image pickup device (22b, 23b) matrix.

## Patentansprüche

1. Endoskopgerät (1), das umfasst:
einen Beleuchtungsabschnitt (21), der ein Subjekt für eine vorbestimmte Blinkperiode abwechselnd mit Speziallicht und Referenzlicht bestrahlt,
ein CMOS Bildaufnahmegerät (22b, 23b), das eine Mehrzahl von Pixelzeilen umfasst, die Licht von dem Subjekt empfangen, das für die vorbestimmte Blinkperiode von dem Beleuchtungsabschnitt (21) mit dem Speziallicht bestrahlt wird und ein Bildaufnahmesignal erzeugt, während einer Leseperiode sequenziell das Bildaufnahmesignal von den Pixelzeilen liest und, nachdem alle Pixel anschließend für die vorbestimmte Blinkperiode Referenzlicht ausgesetzt wurden, während einer weiteren Leseperiode sequenziell das Bildaufnahmesignal von der Mehrzahl von Pixelzeilen liest;
einen Bildwechselfrequenzschalter (53), der eine erste
Bildwechselfrequenzrate und eine zweite Bildwechselfrequenzrate, die höher als die erste Bildwechselfrequenzrate des CMOS-Bildaufnahmegeräts (22b, 23b) ist, festsetzt; und
einen Bildaufnahmegerätetreiber (48), der, wenn die zweite Bildwechselfrequenzrate durch den Bildwechselfrequenzschalter (53) festgesetzt ist, ein Pixelausdünnungslesen durchführt, um die Leseperioden des Bildaufnahmesignals zu verkürzen, indem vorbestimmte Pixel von ausgewählten Zeilen der Mehrzahl von Pixeln, die während der Leseperiode von dem CMOS Bildaufnahmegerät (22b, 23b) gelesen werden sollen, ausgewählt und gelesen werden,
**dadurch gekennzeichnet, dass**
der Bildaufnahmegerätetreiber (48), wenn die zweite Bildwechselfrequenzrate durch den Bildwechselfrequenzschalter (53) festgesetzt ist, die vorbestimmte Blinkperiode festsetzt, die die gleiche ist, wie für die erste Bildwechselfrequenzrate, ohne die vorbestimmte Blinkperiode zu verkürzen.

2. Endoskopgerät (1) gemäß Anspruch 1, bei dem, wenn das Lesen einer Zeile des Bildaufnahmesignals von dem Bildaufnahmegerät (22b, 23b) endet, der Bildaufnahmegerätetreiber (48) Pixel der gelesenen Zeile zurücksetzt.

3. Endoskopgerät (1) gemäß Anspruch 1, bei dem
das CMOS-Bildaufnahmegerät (22b, 23b) ein CMOS Sensor ist, in dem Pixel in einer Matrix angeordnet sind und die Pixel in entsprechenden Zeilen in der Matrix angeordnet sind, und
der Bildaufnahmegerätetreiber (48) für jede Spalte und für jede Zeile der CMOS-Bildaufnahmegeräte- (22b, 23b) Matrix ein Pixelausdünnungslesen durchführt.

## Revendications

1. Appareil endoscopique (1), comprenant :
une section d'éclairage (21) qui irradie de manière alternée un sujet avec une lumière spéciale et une lumière de référence pendant une période de suppression prédéterminée,
un capteur d'image CMOS (22b, 23b) qui comprend une pluralité de lignes de pixels qui reçoivent de la lumière en provenance du sujet qui est irradié avec la lumière spéciale depuis la section d'éclairage (21) pendant la période de suppression prédéterminée et génère un signal de capteur d'image, lit de manière séquentielle le signal de capteur d'image à partir des lignes de pixels, pendant une période de lecture, et après une exposition ultérieure de l'ensemble des pixels à la lumière de référence pendant la période de suppression prédéterminée, lit de manière séquentielle le signal de capteur d'image à partir de la pluralité de lignes de pixels pendant une période de lecture supplémentaire ;
un commutateur de sélection de vitesse de trame (53) qui définit une première vitesse de trame et une seconde vitesse de trame supérieure à la première vitesse de trame du capteur d'image CMOS (22b, 23b) ; et
une commande d'un capteur d'image (48) qui, lorsque la seconde vitesse de trame est définie par le commutateur de sélection de vitesse de trame (53), réalise la lecture de dissipation de pixels pour raccourcir les périodes de lecture du signal de capteur d'image par la sélection et la lecture de pixels prédéterminés à partir des lignes sélectionnées de la pluralité de pixels à lire à partir du capteur d'image CMOS (22b, 23b) pendant la période de lecture, **caractérisé en ce que**
la commande du capteur d'image (48), lorsque la seconde vitesse de trame est définie par la commutation de sélection de vitesse de trame (53), définit la période de suppression prédéterminée qui est la même que pour la première vitesse de trame, sans raccourcir la période de suppression prédéterminée.

2. Appareil endoscopique (1) selon la revendication 1, dans lequel, lorsque la lecture d'une ligne du signal de capteur d'image à partir du capteur d'image (22b, 23b) se termine, la commande du capteur d'image (48) réinitialise les pixels de la ligne lue.

3. Appareil endoscopique (1) selon la revendication 1, dans lequel
le capteur d'image CMOS (22b, 23b) représente un capteur CMOS dans lequel des pixels sont disposés dans une matrice et les pixels sont disposés dans des lignes respectives de la matrice, et
la commande du capteur d'image (48) réalise la lecture de dissipation de pixels pour chaque colonne et pour chaque ligne de la matrice du capteur d'image CMOS (22b, 23b).
